(19) Europäisches Patentamt
European Patent Office
Office européen des brevets

(11) **EP 4 643 852 A1**

(12) **EUROPEAN PATENT APPLICATION**

(43) Date of publication:
05.11.2025 Bulletin 2025/45

(21) Application number: 24382496.8

(22) Date of filing: 06.05.2024

(51) International Patent Classification (IPC):
*A61K 9/48* (2006.01)          *A61K 35/74* (2015.01)

(52) Cooperative Patent Classification (CPC):
A61K 9/4816; A61K 9/4858; A61K 35/74

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 29.04.2024 EP 24382469

(71) Applicant: Mikrobiomik Healthcare Company S.L.
48160 Derio Bizkaia (ES)

(72) Inventors:
• BASTERRA COSSIO, Juan
  48930 GETXO (ES)
• MORALES, Celia
  48500 ABANTO Y ZIERBANA (ES)
• DEL RIO MEDINA, Patricia
  48930 GETXO (ES)

(74) Representative: Galbaian S.Coop.
Garaia Parke Teknologikoa
Goiru kalea 1
20500 Arrasate-Mondragón (ES)

(54) **PHARMACEUTICAL COMPOSITION COMPRISING A PROCESSED FAECAL COMPOSITION**

(57) Pharmaceutical composition in the form of a capsule for oral administration comprising a encapsulated processed faecal composition that comprises faecal microbiota, characterized in that, the processed faecal composition is a powdered lyophilized faecal composition that is encapsulated with a coating shell which is a pH responsive polymer composition and may dissolve or provide a delayed release of the processed faecal composition to the intestine, and wherein the processed faecal composition comprises faecal microbiota comprising colony forming units (CFU) and equal to or less than 1% of human DNA with respect to the total DNA. Method of preparation of the pharmaceutical composition.

FIG. 1

**Description**

TECHNICAL FIELD

The present invention relates to a pharmaceutical composition that comprises a processed faecal composition and to faecal microbiota transplantation.

PRIOR ART

The *Clostridium difficile* bacteria is the main pathogen responsible for diarrhea associated with the previous use of antibiotics in health centers in developed countries, and an increasingly common cause of community diarrhea. Although it usually presents with mild diarrhea, it can also cause serious symptoms such as pseudomembranous colitis, toxic megacolon, septic shock and, in extreme cases, death. The epidemiology of *Clostridium difficile* infection (CDI) has changed dramatically in recent decades, and it has become a major global health problem.

The main risks of CDI are exposure to antibiotics (especially clindamycin, cephalosporins, beta-lactams and fluoroquinolones), prolonged hospitalization, admission to an intensive care unit, physical proximity to infected individuals, advanced age (older age 65), severity of other underlying disease, immunosuppression, poor immune response to CD toxins, nonsurgical gastrointestinal procedures and use of antacids.

The risk of recurrence ranges from 20% after the initial infection to 60% after multiple recurrences, with the recurrence rate being similar in nosocomial or community-acquired infection.

Implantation or administration of human colonic microbiota into the intestine of a patient with CDI is known in the prior art. Infusions of microbiota through a colonoscope, an enema or via a nasojejunal tube in the form of homogenized stool, extracts of homogenized stool are known in the prior art.

The document "Successful Resolution of Recurrent Clostridium Difficile Infection Using Freeze-Dried, Encapsulated Faecal Microbiota; Pragmatic Cohort Study." Am J Gastroenterol 112, no. 6 (Jun 2017): 940-47, describes the use of encapsulated lyophilized faecal microbiota, with a dose of $2.1$-$2.5 \times 10^{11}$ bacteria in 2 or 3 capsules in patients with recurrent CDI. 88% of the treated patients achieved a clinical success, defined as no recurrence of CDI over 2 months.

According to the "Clinical Practice Guideline by the Infectious Diseases Society of America (IDSA) and Society for Healthcare Epidemiology of America (SHEA): 2021 Focused Update Guidelines on Management of *Clostridioides difficile* Infection in Adults", the opinion of the panel is that appropriate antibiotic treatments for at least 2 recurrences (ie, 3 CDI episodes) should be tried prior to offering faecal microbiota transplantation.

DISCLOSURE OF THE INVENTION

The object of the invention is to provide a pharmaceutical composition, as defined in the claims.

A first aspect of the invention relates to a pharmaceutical composition comprising processed faecal composition that comprises faecal microbiota comprising colony forming units (CFU) and a percentage equal to or less than 1% of human DNA with respect to the total DNA. The pharmaceutical composition is in the form of a capsule for oral administration comprising the processed faecal composition, wherein the processed faecal composition is a powdered lyophilized faecal composition. The processed faecal composition is encapsulated with a coating shell which is a pH responsive polymer composition and may dissolve or provide a delayed release of the processed faecal composition to the intestine of the patient, preferably in the large intestine and/or colon.

A second aspect of the invention relates to the pharmaceutical composition defined above for use as a medicament.

Another aspect of the invention relates to the pharmaceutical composition defined above for use in the treatment and/ or prevention of CDI.

Another aspect of the invention relates to a method for the manufacturing of the pharmaceutical composition defined above comprising the following steps:

- a collecting step of stool from a donor or donors, the stool comprising the following characteristics:

    i. absence of liquid, this liquid being selected from non-intrinsic water of the stool, urine and/or blood, and
    ii. normal consistency on the Bristol scale;

- a homogenisation and disaggregation step of the stool of the previous step in isotonic saline solution, preferably in NaCl;
- a filtration step of the homogenised and disaggregated stool in order to remove particles of size $\geq 500$ $\mu$m:
- a centrifugation step of the filtered stool to obtain a sediment that comprises the faecal microbiota;

- an addition step of a solution of a cryoprotectant to the sediment;
- a lyophilization step of the sediment obtained in the previous step;
- a grinding step of the lyophilized sediment in order to obtain a homogeneous powder, preferably with a powder particle diameter between 100 μm and 500 μm; and
- a encapsulation step of the powder in enteric release capsule, preferably in size 0 enteric release capsule, preferably with an average powder content of 250 ± 10% mg/capsule.

The stool or the homogeneous powder comprises colony forming units, preferably the colony forming units comprising bacteroidetes and/or firmicutes, and a percentage equal to or less than 1% of human DNA with respect to the total DNA.

The efficacy of the treatment depends on the quality and characteristics of the pharmaceutical composition and in the pharmaceutic form. In one hand, the processed faecal composition is obtained from donor stool samples. The inventors have realized that the quality of the microbiota could change depending on the health of the donor. It is important to use donor stool or faecal samples that have not human cells, because the presence of human cells in the stool or faecal samples implies that there is a disease or intestinal imbalance in the donor, having a negative impact in the microbiota. The presence of human cells is indirectly measured by quantifying the human DNA in the stool or the processed faecal composition sample. In the other hand, for better efficacy, it is not merely administering microbiota but administering microbiota with colony forming units that will be able to colonize the intestine of the patient.

As shown in example 10 the developed pharmaceutical composition provides a higher clinical benefit in cases of CDI, potentially reducing the need of antibiotics, whereas for recurrent CDI there was a superior efficacy of the invention than the standard treament. Altogether, the invention becomes an effective, safe, easy to administer, accessible and low-risk treatment for CDI.

These and other advantages and features of the invention will become evident in view of the drawings and of the detailed description of the invention.

DESCRIPTION OF THE DRAWINGS

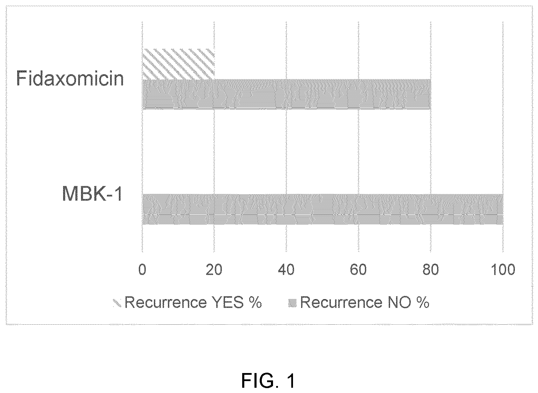

Figure 1 represents a graph of the percentage of recurrences of patients with primary or recurrent CDIs with non-previous antibiotic treatment, using the pharmaceutical composition according to one embodiment of the invention versus fidaxomicin.

DETAILED DISCLOSURE OF THE INVENTION

The pharmaceutical composition developed by the inventors is in the form of a capsule for oral administration comprising processed faecal composition that comprises faecal microbiota. The processed faecal composition is a powdered lyophilized faecal composition. This processed faecal composition is encapsulated with a coating shell which is a pH responsive polymer composition and may dissolve or provide a delayed release of the processed faecal composition to the intestine, preferably in the large intestine and/or colon, and wherein the processed faecal composition comprises faecal microbiota or cells comprising viable colony forming units (CFU) and a percentage equal to or less than 1% of human DNA with respect to the total DNA present in the processed faecal composition, preferably, between 0% and 1% of human DNA with respect to the total DNA present in the processed faecal composition. The human DNA comes from human cells from the donor, considered as impurities of the processed faecal composition, and it is calculated according to the following formula:

$$\% \text{ Human DNA} = 100 \times \text{quantity of Human DNA (ng)} / \text{quantity of Total DNA (ng)}$$

In the context of the invention, "viable" means possessing the ability to multiply.

In the context of the invention, "colony forming units" (CFU) refers to an estimate of the number of viable microorganism cells in a given sample. In the context of the invention, the viability of cfu is monitored as a function of forming colonies of microorganism.

In the context of the invention, "the processed faecal composition" means the faecal or stool sample that has been processed to obtain a concentrated faecal microbiota, for example by filtration or the steps described in the method for the manufacturing of the pharmaceutical composition of the invention. The processed faecal composition is also known as intestinal microbiota.

In the context of the invention, "microbiota" refers to a community of microbes that live in or on a subject's body, both sustainably and transiently, including eukaryotes, archaea, bacteria, and viruses (including bacterial viruses (i.e., phage)). A "faecal microbiota" refers to a community of microbes present in a subject's faeces or stool.

In the context of the invention, unless indicated otherwise, percent concentration refers to weight-to-weight percent (% w/w).

In a particular embodiment, the processed faecal composition comprises at least 50% of colony forming units per grame of processed faecal composition respect to the total microorganism present in the processed faecal composition, preferably being the colony forming units of bacteroidetes and/or firmicutes. In a preferred embodiment, the at least 50% of colony forming units per grame of processed faecal composition comprises between 20 and 80% of bacteroidetes and/or between 20 and 80% of firmicutes.

The pharmaceutical composition comprises the processed faecal microbiota with pharmaceutically acceptable excipients or carriers, in a subject in need of that treatment and/or prevention, including a human being. The person skilled in the art can determine what additional components can be used and if they are necessary, many of them being commonly used in pharmaceutical compositions.

The expression "pharmaceutically acceptable excipients or carriers" refers to pharmaceutically acceptable materials, composition or vehicles. Each component must be pharmaceutically acceptable in the sense of being compatible with the other ingredients of the pharmaceutical composition. It must also be suitable for use in contact with human and animal tissues or organs without excessive toxicity, irritation, allergic reaction, immunogenicity or other problems or complications in accordance with a reasonable risk-benefit ratio.

In a particular embodiment, the processed faecal composition of the pharmaceutical composition comprises faecal microbiota, a pharmaceutically acceptable salt and a pharmaceutically acceptable cryoprotectant, wherein the faecal microbiota is between 30 and 75% by weight, the salt is between 1 and 20% by weight and the cryoprotectant is between 20 and 50% by weight.

In a preferred embodiment, the processed faecal composition of the pharmaceutical composition consists of faecal microbiota, a pharmaceutically acceptable salt and a pharmaceutically acceptable cryoprotectant, wherein the faecal microbiota is between 30 and 75% by weight, the salt is between 1 and 20% by weight and the cryoprotectant is between 20 and 50% by weight. This composition, itself, implies the use of non-toxic excipients that promote better treatment.

Regarding the pharmaceutically acceptable salt, there are no limitations on the types of salts that can be used, as long as they are pharmaceutically acceptable for use for therapeutic purposes. In a preferred embodiment, the compound of the invention is preferably a chloride salt, preferably sodium chloride. The sodium chloride helps to maintain the cell osmolarity, therefore, the viability of the microbiota during the production of the pharmaceutical composition of the invention.

Regarding the pharmaceutically acceptable cryoprotectant, this cryoprotectant can be selected, without limitation, from trehalose, lactose, maltose, sucrose or any other saccharide or not, which acts as a cryoprotectant and/or lyoprotectant, preferably being trehalosa. The cryoprotectant favors microbiota stabilization during the freezing process of the lyophilization process. In a preferred embodiment, the faecal composition comprises trehalose as a cryoprotectant.

In the context of the invention, a "cryoprotectant" refers to a substance that is added to a formulation in order to protect an active ingredient during freezing.

Lyophilization allows obtaining a dry powder containing the faecal microbiota, which provides it with greater stability than faecal microbiota in suspension, since it prevents microbiota degradation due to the fact that removing humidity, the proliferation of microorganism of the processed faecal sample is minimized or avoided. In one embodiment, the powdered faecal composition comprises powder particle diameter or size between 100 $\mu$m and 500 $\mu$m. The diameter or the size is measured by the sieving method.

In a preferred embodiment, the weight of the processed faecal composition is 250 mg per capsule, preferably with a concentration of faecal microbiota is between $0,5 \times 10^{11}$ and $0,6 \times 10^{11}$ cells per capsule.

Regarding the encapsulation, in a preferred embodiment, the processed faecal composition is single encapsulated with one coating shell, the one coating shell comprising polymers selected from hypromellose and/or acetate succinate and/or water-insoluble (meth)acrylate copolymer, and/or hydroxypropyl methyl propylcellulose and/or their derivatives and/or mixtures thereof, preferably, hypromellose. This encapsulation has been demonstrated a delayed release of the processed faecal composition to the intestine, after the oral administration of the capsule.

For improving the stability of the capsule, in a preferred embodiment, the pharmaceutical composition is packaged in a high-density polyethylene container with a tamper-proof cap, the container being packaged within a three-layer pouch of polyethylene, aluminium and low-density polystyrene which is airtight. This packaging has demonstrated a better stability of the pharmaceutical composition. With this packaging an anaerobic condition is created and minimizes and/avoids microbiota proliferation.

Another aspect of the invention relates to the pharmaceutical composition defined above for use as a medicament.

Another aspect of the invention relates to the pharmaceutical composition defined above for use in the treatment and/or prevention of infections caused by *Clostridium difficile.*

In the context of the invention, the term "prevention or treatment" means the administration of the pharmaceutical composition according to the invention to preserve the health of a patient who is suffering or who is at risk of suffering a infection described above. Said terms also include the administration of the pharmaceutical composition according to the invention to prevent, improve, alleviate or eliminate one or more symptoms associated with CDI. In the context of this invention, the term "improve" is understood to mean any improvement in the situation of the treated patient, that is either a subjective improvement (how the patient feels) or an objective improvement (measured parameters).

In a preferred embodiment, the faecal microbiota is provided at a single dose of $\geq 2 \times 10^{11}$ cells of microbiota, preferably between $2 \times 10^{11}$ and $2,4 \times 10^{11}$ cells to a patient with a CDI, preferably, to a patient with a primary infection of CDI that has not been treated with an antibiotic before. As it can be seen in example 10, the effectiveness of the treatment is better in those patients that have not been treated with an antibiotic before. In a preferred embodiment, the dose is provided with 4 capsules that add up a single dose of $2 \times 10^{11}$ and $2,4 \times 10^{11}$ cells. The capsule is for oral administration within a non-aqueous medium or vehicle, in order to minimize or avoid the dissolution of the capsule out of the intestine and avoid the degradation of the faecal microbiota before reaching the intestine.

Another aspect of the invention relates to a method of preparation or manufacturing of a pharmaceutical composition of processed faecal composition, preferably, of a pharmaceutical composition of the invention, comprising the following steps:

a) collecting step of stool from a donor or donors, the stool comprising the following characteristics:

i. absence of liquid, this liquid being selected from non-intrinsic water of the stool, urine and/or blood, and
ii. normal consistency on the Bristol scale;

b) homogenisation and disaggregation step of the stool of the previous step in an isotonic saline solution, preferably in NaCl;
c) filtration step of the homogenised and disaggregated stool in order to remove particles of size $\geq 500$ $\mu$m:
d) centrifugation step of the filtered stool of the previous step to obtain a sediment that comprises the faecal microbiota;
e) addition step of a solution of a cryoprotectant to the sediment, preferably 5% w/v of cryoprotectant in saline solution, in a 1:1 ratio to the volume of sediment;
f) lyophilization step of the sediment obtained in the previous step;
g) grinding step of the lyophilized sediment in order to obtain a homogeneous powder, preferably with a powder particle diameter between 100 $\mu$m and 500 $\mu$m; and
h) encapsulation step of the powder in enteric release capsule, preferably in size 0 enteric release capsule, preferably with an average powder content of 250 $\pm$ 10% mg/capsule;

wherein the the stool or the homogeneous powder to be encapsulated or encapsulated comprises colony forming units, preferably the colony forming units comprising bacteroidetes and/or firmicutes, and a percentage equal to or less than 1% of human DNA with respect to the total DNA.

In the context of the invention, "non intrinsic water of the stool" means any water that exceeds from the water normally present in stool. This exceeding water is visually perceptible or noticeable.

In the context of the invention, "lyophilization" refers to the process of drying a material by first freezing it and then encouraging the ice within it to sublimate in a vacuum environment.

Regarding the Bristol stool scale, it is a visual table used in medicine, intended to classify the shape of human stool into seven groups. It was developed by Ken W. Heaton and Stephen J. Lewis at the University of Bristol and was first published in the Scandinavian Journal of Gastroenterology in 1997 (Lewis SJ, Heaton KW (1997). «Stool form scale as a useful guide to intestinal transit time». Scandinavian Journal of Gastroenterology, 32(9): 920-4). In the context of the invention normal consistency apply to type 2/3 to type 5 of the Bristol scale.

As a result of any step b to g, a processed faecal composition is obtained. In a preferred embodiment, the processed faecal composition is obtained as a result of step b to g. The processed faecal composition may be also used in another pharmaceutical form, for example, resuspending processed faecal powder and in a liquid format for tube administration.

As a quality control measure, the method may comprise a determination or quantification step of the percentage of human cells as impurities. In a preferred embodiment, the determination or quantification step of the percentage of human cells as impurities comprises quantifying the % of human DNA with respect to the total DNA present in the stool to be processed or in the processed faecal composition, according to the following formula:

$$\% \text{ Human DNA} = 100 \times \text{quantity of Human DNA (ng) / quantity of Total DNA (ng)}$$

For calculating the % Human DNA, in a preferred embodiment, DNA is extracted from the stool or at least one sample of the processed faecal composition, preferably from 1gr of stool or at least one sample of the processed faecal composition, preferably from the stool sample obtained in step c). Therefore, the % human DNA may be calculated per gram of stool or processed faecal composition. The total DNA is quantified preferably by spectrofluorometry and, the human DNA present is quantified, preferably by quantitative PCR, using probes against a human DNA standard with a known quantity of human DNA. In this way, the percentage of human DNA respect to the total DNA detected in the sample is calculated.

In one embodiment, donors are healthy donors, understood as donors that are eligible according to any faecal microbiota transplant guidelines in clinical practice such as the British Society of Gastroenterology (BSG) and Healthcare Infection Society (HIS) guidelines (Mullish BH, Quraishi MN, Segal JP, et al. Gut 2018;67:1920-1941) or the "International consensus conference on stool banking for faecal microbiota transplantation in clinical practice" (Cammarota G, Ianiro G, Kelly CR, et al. Gut 2019;68:2111-2121).

The method of preparation of the invention allows the reproducibility of batches, the stability and viability of the faecal microbiota during the storage and oral administration of the pharmaceutical composition.

Regarding the method of preparation of the pharmaceutical composition of the invention, the technical features described in the various embodiments of the pharmaceutical composition of the invention apply to this aspect of the invention giving rise to its various embodiments.

## Examples

Some illustrative examples which clearly show the features and advantages of the invention are described below. However, these examples must not be interpreted as limiting the object of the invention as defined in the claims.

Example 1: Preparation of the pharmaceutical composition according to one embodiment of the invention:

The following method has been followed for the preparation of different batches of pharmaceutical composition according to one embodiment of the invention:

a) Obtaining of stool from healthy donor or donors, the stool having the following characteristics:

- absence of liquid which consists of water, urine and/or blood, and
- normal consistency on the Bristol scale.

b) Homogenisation and disaggregation of the stool of the previous step in NaCl solution.
c) Filtration of the homogenised and disaggregated of the stool in order to remove particles of size $\geq 500$ $\mu$m.
d) Centrifugation of the filtered stool of the previous step to obtain the sediment that comprises the faecal microbiota.
e) Addition of a solution of a cryoprotectant (trehalose), in 5% of solution of cryoprotectant in saline in a 1:1 ratio to the volume of sediment.
f) Deep freezing step at temperature $\pm$ -80°C and lyophilization step of the previous sediment.
g) Grinding step of the lyophilized sediment in order to obtain a homogeneous powder with a powder particle diameter between 100 $\mu$m and 500 $\mu$m; and
h) Encapsulation step of the powder in gastro-resistant capsule with an average powder content of 250 $\pm$ 10% mg/capsule.

The composition of the powder obtained after freeze-drying of the batches manufactured was determined by weighing difference and the results vary in the following ranges by weight:

Faecal microbiota from 34.37% to 72.84%.
Salt (NaCl) from 4.13% to 17.37%.
Cryoprotectant from 23.03% to 48.26%.

Example 2: Determination of the percentage of human cells by quantifying the % of human DNA with respect to the total DNA present in a sample:

For this purpose DNA is extracted from a sample of the homogenised stool sample of each batch, the total DNA has been quantified by spectrofluorometry and the human DNA present has been quantified by quantitative PCR, using taqman™ probes, against a human DNA standard with a known quantity. In this way, the percentage of human DNA respect to the total DNA detected in the sample has been established.

The results obtained with the analysed samples taken from stage c) of example 1 in terms of the percentage of human cells considered as impurities, expressed as % of human DNA respect total DNA detected in the sample, vary between 0,0047 - 0,8480%. Samples obtained from 73 batches according to example 1 have been analysed.

Example 3: Determination of the number of total microorganisms by direct microscopic counting or automated counting by automated counter, after labelling the cells with fluorochromes:

The results obtained with the analysed samples of homogenised stool, regarding the Logarithm of the number of total microorganisms / g of starting material (taken from stage c) of example 1) varied between 11,15 and 12,32.

Example 4: Determination of the number of colony forming units (CFU) by culturing aerobic and anaerobic microorganisms in general media.

The results obtained in terms of the percentage of colony forming units (CFU's) per gram of homogenised stool with respect to the number of total microorganisms per gram of homogenised stool, varied between 77.82 and 92.24%.

Example 5: Analysis of bacterial composition:

The bacterial composition of each donor's stool is characterised to show: 20-80% Bacteroidetes and 20-80% Firmicutes.

For this purpose, the stool sample is collected in a vial containing a DNA stabiliser solution. DNA is extracted from this sample, amplified, and subjected to mass sequencing of the 16S gene. The sequencing results obtained is compared with bibliographic databases to determine the species present in the sample. These species will be classified in their corresponding Phylums.

The Phylum classification of stool samples were:

| Donor | % Bacteroidetes | % Firmicutes |
|-------|-----------------|--------------|
| 1 | 49.63 | 45.35 |
| 2 | 49.05 | 37.79 |
| 3 | 52.39 | 40.95 |
| 4 | 29.49 | 62.65 |
| 5 | 55.10 | 39.20 |
| 6 | 57.62 | 39.73 |
| 7 | 72.83 | 23.75 |
| 8 | 41.40 | 53.43 |
| 9 | 34.10 | 61.72 |
| 10 | 43.65 | 45.11 |

Example 6: Determination of the number of total microorganisms by direct microscopic counting or automated counting by automated counter, after labelling the cells with fluorochromes:

The results obtained regarding the number of total microorganisms / g of powdered lyophilized faecal composition, i.e. number of total microorganisms per dose, vary between $1{,}59 \times 10^{12}$ - $9{,}72 \times 10^{12}$ cells.

This translates into a total number of microorganisms of between $3{,}82 \times 10^{11}$ - $3{,}49 \times 10^{12}$ per capsule of final product with $250 \pm 10\%$ mg of /capsule.

Example 7: Determination of the number of colony forming units (CFU) by culturing aerobic and anaerobic microorganisms in general media of processed faecal composition:

The results obtained in terms of the percentage of colony forming units (CFU's) / g of processed faecal composition that have been lyophilized and encapsulated, with respect to the number of total microorganisms / g of processed faecal composition vary between 57,35 - 82,26%.

Example 8: Stability study

In the stability study carried out with the pharmaceutical composition in the form of a capsule as described above, the product has been tested after storage for 0, 3, 4 and 6 months under refrigerated conditions (2-8°C).

The analyses carried out during each stability time are as follows: Quantification of total microorganisms and quantification of viable microorganisms.

The maximum storage period of the product (Expiry Date) has been determined when it reaches the established activity loss limits:

- Loss Total microorganisms 10% $Log_{10}$ No. of total microorganisms at time=t
- Loss Viable microorganisms 20% $Log_{10}$ No. of viable microorganisms at time=t

The results obtained show that after 6 months of storage of the product, the maximum loss of total microorganisms with respect to $Log_{10}$ No. total microorganisms at time=6 month is 3,14% and the maximum loss of viable microorganisms with respect to $Log_{10}$ No. total microorganisms at time=6 month is 19,74%. Therefore, the latter result has reached the maximum limit and the expiry date has been set, at least 6 months. However, the product still has activity, therefore, the product could still be administrated.

Example 9: Release of the microbiota in the intestine

Because the efficacy of the drug is not only about the amount of microbiota administered, but also about whether the administered microbiota manages to reach and colonise the patient's gut. Inventors have conducted dynamic *in vitro* tests simulating gastrointestinal digestion to demonstrate that the microbiota reaches the gut and most of its contents are released in the gut, rather than in the stomach.

For these tests, an *in vitro* Dynamic Digester has been used to reproduce the human stomach and intestinal digestion process. The *in vitro* Dynamic Digester consists of two compartments simulating the stomach and small intestine. Each compartment consists of a double-jacketed reactor (surrounded by water to maintain a constant temperature of 37°C). In the digestion chambers, both in the stomach section and in the intestinal section, although reactions are generated, the pH is continuously controlled by secretions of hydrochloric acid in the stomach and sodium bicarbonate in the small intestine. In addition, pepsin solution and gastric lipase are introduced into the stomach digestion chamber. During dynamic digestion, gastric and intestinal transit is gradually simulated by opening and closing valves. During the intestinal digestion process, different enzymatic and chemical solutions are introduced at constant flows. Throughout the digestion process, samples are taken for counting total and viable microorganisms.

The results of these tests have shown that there is no release of the capsule contents in the gastric phase in any of the tests performed. At 7-12 minutes after the start of the intestinal phase, the capsules began to show signs of dissolution. After 30 minutes of the intestinal phase, it was quantified that the number of total microorganisms released into the intestine was between 90,58 - 100% of those found in the capsule. Of these microorganisms released into the gut, the inventors have quantified that at least 52,65 - 84,06% are able to colonise the gut (colony forming units).

Example 10: Clinical study in humans

A clinical study has been carried out with the following characteristics:

- Type of clinical study:randomised, controlled and open label study
- Conditions fulfilled for recruitment of the patient:

Diagnosis criteria:
Patients were included in the study following presentation of an episode of diarrhoea (≥3 stools/24 hours), and confirmation of CD toxin A and/or B in faeces, by a direct toxin detection test or by the Polymerase chain reaction (PCR) technique for the detection of toxin/s producing genes. In addition, they had to meet the inclusion and not meet any of the exclusion criteria.

Inclusion criteria:

- Patients of both genders, over 18 years.
- Patients that undergo an episode of CD infection (either the first episode or subsequent recurrences).
- Presence of an episode of diarrhoea defined as ≥3 stools/24 hours, at the beginning of the episode.
- Confirmation of the presence of CD toxin A and/or B in faeces, by a direct toxin detection test or by the PCR technique for the detection of toxin/s produci genes, at the start of the episode that is going to be treated in the clinical trial (the toxin test must be positive within 7 days prior to the enrolment of the patient in the trial).

Exclusion criteria:

- Previous faecal microbiota transfer.
- Transplanted patients, except those with a solid organ transplant of more than 2 years, with good organ function.
- Absolute neutrophil count <500 cells /$\mu$L at the time of the enrolment in the study.
- Pregnancy, breastfeeding, or pregnancy intentions over the course of the study.
- Active treatment with bile acid sequestrants (for instance: cholestyramine).
- Patients positive for the human immunodeficiency virus (HIV) except those with lymphocytes T CD4 count > 200 cells/$\mu$L and viral load less than 20 copies.
- Swallowing dysfunction or no oral motor coordination.
- Patient admitted in an intensive care unit or expected to be admitted in an intensive care unit due to serious illness.
- History of significant medical conditions that, in the opinion of the investigator, would not allow an adequate evaluation or follow-up of the patient.

- Administered pharmaceutical composition:
  Capsules elaborated according to the method of the invention and example 1 (MBK-1).

Dose:

The treatment regimen was 4 capsules in a single dose ($\geq$2.1-2.5 $\times$ $10^{11}$ microorganisms). The timing of intake was left to the discretion of the doctor and was taken orally as a single dose and with empty stomach. If the patient had difficulty swallowing, a gel or gelled water could be used to facilitate the swallowing process.
The duration of treatment was different according to the assigned group:

- Patients in the experimental group received capsules MBK-1: a single dose of 4 capsules.
- Patients in the control group received fidaxomicin: a dose of 200 mg (one tablet), administered twice a day (every 12 hours) for 10 days.
- Result:
  The results in this study have suggested greater clinical benefit of treatment with the pharmaceutical composition of the invention (MBK-1) when compared with fidaxomicin when it is used as the first choice for treatment of the current episode as shown in the following tables, both for primary CDI episodes and recurrent CDI episodes, even in some cases without prior antibiotic treatment at all (table 2).

TABLE 1: Count and proportions of recurrence in each group

| | Recurrence | | | | |
| | No | | Yes | | |
| Treatment | Number | % | Number | % | Proportion* |
|---|---|---|---|---|---|
| MBK-1 | 33 | 89,19 | 4 | 10,81 | 0,8919 |
| Fidaxomicin | 31 | 77,5 | 9 | 22,5 | 0,775 |
| *Proportion=No recurrence/Total patients in each group | | | | | |

TABLE 2: Count and proportions: non previous antibiotic treatment subgroup

| | Recurrence | | | | |
| | No | | Yes | | |
| Treatment | Number | % | Number | % | Proportion* |
|---|---|---|---|---|---|
| MBK-1 | 17 | 100 | 0 | 0 | 1 |

(continued)

| | Recurrence | | | | |
|---|---|---|---|---|---|
| | No | | Yes | | Proportion* |
| Treatment | Number | % | Number | % | |
| Fidaxomicin | 12 | 80 | 3 | 20 | 0,8 |
| *Proportion=No recurrence/Total patients in each group | | | | | |

Recurrence means that the treated patient has had a recurrent CDI episode within 8 weeks after the onset of the previous episode.

The clinical benefit stems from different reasons in primary and recurrent episodes of CDI. For the primary episodes the benefit resides in the lesser use of antibiotics and therefore, lower risk of antibiotic resistance emergence. For the recurrent episodes of CDI though, there is direct clinical benefit due to a superior efficacy of invention against fidaxomicin.

Although the use of the pharmaceutical composition of the invention as first line of treatment contradicts current guidelines for the management of CDI, which recommend considering the use of faecal microbiota transplantation (FMT) only for recurrent episodes, it is supported by the increasing probability of recurrence with further episodes of CDI and therefore increasing rounds of antibiotic treatment.

Lastly, in regard to safety, it is to note that there were no side adverse effects (SAEs) related to the pharmaceutical composition of the invention, and that no unexpected adverse effects (AEs) were reported in the study. Therefore, the treatment with the composition of the invention can be considered safe, with a low risk profile.

**Claims**

1. Pharmaceutical composition in the form of a capsule for oral administration, comprising an encapsulated processed faecal composition that comprises faecal microbiota, **characterized in that** the processed faecal composition is a powdered lyophilized faecal composition that is encapsulated with a coating shell which is a pH responsive polymer composition and may dissolve or provide a delayed release of the processed faecal composition to the intestine, and wherein the processed faecal composition comprises faecal microbiota comprising colony forming units (CFU) and a percentage equal to or less than 1% of human DNA with respect to the total DNA.

2. Pharmaceutical composition according to claim 1, wherein the microbiota comprises at least 50% of colony forming units/g of processed faecal composition, the colony forming units preferably comprising between 20 and 80% of bacteroidetes and between 20 and 80% of firmicutes.

3. Pharmaceutical composition according to claim 1 or 2, wherein the processed faecal composition comprises faecal microbiota, a pharmaceutically acceptable salt and a pharmaceutically acceptable cryoprotectant, wherein the faecal microbiota is between 30 and 75% by weight, the salt is between 1 and 20% by weight and the cryoprotectant is between 20 and 50% by weight with respect to the processed faecal composition.

4. Pharmaceutical composition according to any of the preceding claims, wherein the powdered faecal composition comprises a powder particle diameter between 100 $\mu$m and 500 $\mu$m.

5. Pharmaceutical composition according to any of the preceding claims, wherein the weight of the processed faecal composition is 250 mg per capsule.

6. Pharmaceutical composition according to any of the preceding claims, wherein the concentration of faecal microbiota is between $0,5 \times 10^{11}$ and $0,6 \times 10^{11}$ cells per capsule.

7. Pharmaceutical composition according to any of the preceding claims, wherein the processed faecal composition is single encapsulated with one coating shell, the one coating shell comprising polymers selected from hypromellose and/or hypromellose-acetate succinate and/or water-insoluble (meth)acrylate copolymer, and/or hydroxypropyl methyl propylcellulose and/or their derivatives and/or mixtures thereof.

8. Pharmaceutical composition according to any of the preceding claims, wherein the pharmaceutical composition is packaged in a high-density polyethylene container with a tamper-proof cap, the container being packaged within a three-layer pouch of polyethylene, aluminum and low-density polystyrene which is airtight.

9. Pharmaceutical composition according to any of the preceding claims, for use as a medicament.

10. Pharmaceutical composition according to any of the preceding claims, for use in the treatment and/or prevention of infections caused by *Clostridium difficile.*

11. Pharmaceutical composition according to claim 10, wherein the faecal microbiota is provided at a single dose of $2 \times 10^{11}$ and $2,4 \times 10^{11}$ cells.

12. Pharmaceutical composition according to claim 10 or 11, wherein the treatment comprises administering the pharmaceutical composition to a patient with a primary infection that has not been treated with an antibiotic before the treatment with the pharmaceutical composition.

13. Pharmaceutical composition according to any of claims 10 to 12, wherein the treatment comprises oral administration of the pharmaceutical composition within a non-aqueous medium or vehicle.

14. Method of preparation of the pharmaceutical composition according to any of the preceding claims, the method comprising the following steps:

   - a collecting step of stool from a donor or donors, the stool comprising the following characteristics:

      i. absence of liquid, this liquid being selected from non-intrinsic water of the stool, urine and/or blood, and
      ii. normal consistency on the Bristol scale;

   - a homogenisation and disaggregation step of the stool of the previous step in isotonic saline solution, preferably in NaCl;
   - a filtration step of the homogenised and disaggregated stool in order to remove particles of size $\geq 500\mu m$:
   - a centrifugation step of the filtered stool to obtain a sediment that comprises the faecal microbiota;
   - an addition step of a solution of a cryoprotectant to the sediment;
   - a lyophilization step of the sediment obtained in the previous step;
   - a grinding step of the lyophilized sediment in order to obtain a homogeneous powder, preferably with a powder particle diameter between 100 $\mu m$ and 500 $\mu m$; and
   - a encapsulation step of the powder in enteric release capsule, preferably in size 0 enteric release capsule, preferably with an average powder content of 250 $\pm$ 10% mg/capsule;

   wherein the stool or the homogeneous powder comprises colony forming units, the colony forming units preferably comprising bacteroidetes and/or firmicutes, and a percentage equal to or less than 1% of human DNA with respect to the total DNA.

15. Method according to claim 14, wherein the method comprises a determination or quantification step of human DNA according to the following formula in a sample obtained from the stool or the homogeneous powder:

$$\% \text{ human DNA} = 100 \times \text{quantity of Human DNA (ng)} / \text{quantity of Total DNA (ng)}$$

FIG. 1

Europäisches
Patentamt

European
Patent Office

Office européen
des brevets

**EUROPEAN SEARCH REPORT**

Application Number

EP 24 38 2496

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 110 721 202 A (THIRD PEOPLES HOSPITAL OF SHENZHEN) 24 January 2020 (2020-01-24) | 1-3,6, 9-14 | INV. A61K9/48 |
| Y | * paragraph [0027] - paragraph [0037] * <br> * claims 1-7 * | 1-15 | A61K35/74 |
| X | WO 2011/033310 A1 (MARTIN WILLIAM JOHN [GB]) 24 March 2011 (2011-03-24) | 1,7,9,10 | |
| Y | * claims 1-14 * <br> * page 20 - page 21 * <br> * tables 1, 2 * | 1-15 | |
| X | WO 2012/016287 A2 (BORODY THOMAS J [AU]) 9 February 2012 (2012-02-09) | 1,9,10 | |
| Y | * example 1 * <br> * page 26, line 20 - line 26 * | 1-15 | |

| TECHNICAL FIELDS SEARCHED (IPC) |
|---|
| A61K |

The present search report has been drawn up for all claims

| Place of search | Date of completion of the search | Examiner |
|---|---|---|
| Munich | 15 October 2024 | Sindel, Ulrike |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

.............................................................................

& : member of the same patent family, corresponding
  document

EPO FORM 1503 03.82 (P04C01)

**EP 4 643 852 A1**

## ANNEX TO THE EUROPEAN SEARCH REPORT
## ON EUROPEAN PATENT APPLICATION NO.

EP 24 38 2496

15-10-2024

| Patent document cited in search report | | Publication date | Patent family member(s) | | Publication date |
|---|---|---|---|---|---|
| CN 110721202 | A | 24-01-2020 | NONE | | |
| WO 2011033310 | A1 | 24-03-2011 | NONE | | |
| WO 2012016287 | A2 | 09-02-2012 | AU | 2011286165 A1 | 07-03-2013 |
| | | | BR | 112013002667 A2 | 31-05-2016 |
| | | | CA | 2807242 A1 | 09-02-2012 |
| | | | CN | 103124559 A | 29-05-2013 |
| | | | EP | 2600877 A2 | 12-06-2013 |
| | | | EP | 3311825 A1 | 25-04-2018 |
| | | | EP | 3424515 A2 | 09-01-2019 |
| | | | EP | 3701958 A1 | 02-09-2020 |
| | | | IL | 224527 A | 30-11-2016 |
| | | | JP | 6606689 B2 | 20-11-2019 |
| | | | JP | 2013537531 A | 03-10-2013 |
| | | | JP | 2016199581 A | 01-12-2016 |
| | | | JP | 2018021052 A | 08-02-2018 |
| | | | JP | 2023145530 A | 11-10-2023 |
| | | | KR | 20130098320 A | 04-09-2013 |
| | | | KR | 20170075809 A | 03-07-2017 |
| | | | KR | 20190114036 A | 08-10-2019 |
| | | | MX | 344831 B | 04-01-2017 |
| | | | NZ | 607043 A | 29-05-2015 |
| | | | NZ | 618935 A | 28-03-2014 |
| | | | RU | 2013109251 A | 10-09-2014 |
| | | | SG | 187713 A1 | 28-03-2013 |
| | | | US | 2013195804 A1 | 01-08-2013 |
| | | | US | 2016296568 A1 | 13-10-2016 |
| | | | US | 2018036351 A1 | 08-02-2018 |
| | | | US | 2018228849 A1 | 16-08-2018 |
| | | | US | 2018256652 A1 | 13-09-2018 |
| | | | US | 2018369295 A1 | 27-12-2018 |
| | | | US | 2019015460 A1 | 17-01-2019 |
| | | | US | 2019015461 A1 | 17-01-2019 |
| | | | US | 2019015462 A1 | 17-01-2019 |
| | | | US | 2019046589 A1 | 14-02-2019 |
| | | | US | 2019216860 A1 | 18-07-2019 |
| | | | US | 2019282631 A1 | 19-09-2019 |
| | | | US | 2019343897 A1 | 14-11-2019 |
| | | | US | 2019381114 A1 | 19-12-2019 |
| | | | US | 2020108104 A1 | 09-04-2020 |
| | | | US | 2020289584 A1 | 17-09-2020 |
| | | | US | 2020397833 A1 | 24-12-2020 |
| | | | US | 2020397834 A1 | 24-12-2020 |
| | | | US | 2021052673 A1 | 25-02-2021 |
| | | | US | 2021205375 A1 | 08-07-2021 |

EPO FORM P0459

page 1 of 2

## ANNEX TO THE EUROPEAN SEARCH REPORT
## ON EUROPEAN PATENT APPLICATION NO.

EP 24 38 2496

This annex lists the patent family members relating to the patent documents cited in the above-mentioned European search report.
The members are as contained in the European Patent Office EDP file on
The European Patent Office is in no way liable for these particulars which are merely given for the purpose of information.

15-10-2024

| Patent document cited in search report | Publication date | Patent family member(s) | | Publication date |
|---|---|---|---|---|
| | | US | 2022023356 A1 | 27-01-2022 |
| | | US | 2022175853 A1 | 09-06-2022 |
| | | US | 2022257672 A1 | 18-08-2022 |
| | | US | 2023210914 A1 | 06-07-2023 |
| | | US | 2023210915 A1 | 06-07-2023 |
| | | US | 2024100101 A1 | 28-03-2024 |
| | | WO | 2012016287 A2 | 09-02-2012 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Successful Resolution of Recurrent Clostridium Difficile Infection Using Freeze-Dried, Encapsulated Faecal Microbiota; Pragmatic Cohort Study.. *Am J Gastroenterol*, June 2017, vol. 112 (6), 940-47
- **KEN W. HEATON** ; **STEPHEN J. LEWIS**. *Scandinavian Journal of Gastroenterology*, 1997

- **LEWIS SJ** ; **HEATON KW**. Stool form scale as a useful guide to intestinal transit time. *Scandinavian Journal of Gastroenterology*, 1997, vol. 32 (9), 920-4
- **MULLISH BH** ; **QURAISHI MN** ; **SEGAL JP et al.** *Gut*, 2018, vol. 67, 1920-1941
- **CAMMAROTA G** ; **LANIRO G** ; **KELLY CR et al.** *Gut*, 2019, vol. 68, 2111-2121